(19) Europäisches Patentamt

European Patent Office

Office européen des brevets

(11) **EP 1 177 160 B1**

(12) **EUROPÄISCHE PATENTSCHRIFT**

(45) Veröffentlichungstag und Bekanntmachung des Hinweises auf die Patenterteilung:
**23.08.2006 Patentblatt 2006/34**

(21) Anmeldenummer: **00935010.9**

(22) Anmeldetag: **11.05.2000**

(51) Int Cl.:
**C07C 2/08** (2006.01)

(86) Internationale Anmeldenummer:
**PCT/EP2000/004286**

(87) Internationale Veröffentlichungsnummer:
**WO 2000/069795 (23.11.2000 Gazette 2000/47)**

(54) **VERFAHREN ZUR OLIGOMERISIERUNG VON C2- BIS C8-OLEFINEN**

METHOD FOR THE OLIGOMERISATION OF C2-C8-OLEFINS

PROCEDE D'OLIGOMERISATION D'OLEFINES C2 A C8

(84) Benannte Vertragsstaaten:
**BE DE FR NL**

(30) Priorität: **12.05.1999 DE 19922038**

(43) Veröffentlichungstag der Anmeldung:
**06.02.2002 Patentblatt 2002/06**

(73) Patentinhaber: **BASF Aktiengesellschaft**
**67056 Ludwigshafen (DE)**

(72) Erfinder:
• **SCHULZ, Ralf**
**D-67346 Speyer (DE)**

• **WALTER, Marc**
**D-67227 Frankenthal (DE)**
• **NEUMANN, Hans-Peter**
**D-67067 Ludwigshafen (DE)**
• **BROX, Wolfgang**
**D-69118 Heidelberg (DE)**

(74) Vertreter: **Kinzebach, Werner et al**
**Ludwigsplatz 4**
**67059 Ludwigshafen (DE)**

(56) Entgegenhaltungen:
**WO-A-99/25668           DE-A- 4 339 713**
**US-A- 4 942 021**

**Beschreibung**

Die vorliegende Erfindung betrifft ein Verfahren zur Oligomerisierung von $C_2$- bis $C_8$-Olefinen in mehreren aufeinanderfolgenden Reaktionszonen.

Olefine mit 2 bis 8 Kohlenstoffatomen oder deren Gemische stehen in großen Mengen sowohl aus FCC-Anlagen (Fluidized Catalyst Cracking) als auch aus Steamcrackern zur Verfügung. Es ist bekannt, die $C_4$-Fraktion, d.h. ein im Wesentlichen aus Butenen und Butanen bestehendes Gemisch, gegebenenfalls nach Abtrennung des iso-Butens, zur Herstellung von Oligomeren, insbesondere von Octenen und Dodecenen, zu verwenden. Sowohl die Octene als auch die Dodecene können nach der Hydroformylierung und anschließender Hydrierung zu den entsprechenden Alkoholen z.B. zur Herstellung von Weichmachern oder Tensiden verwendet werden.

Die Oligomerisierung wird großtechnisch entweder unter homogener oder heterogener Katalyse durchgeführt. Das homogen katalysierte Verfahren weist den Nachteil auf, dass der Katalysator vom Reaktionsgemisch getrennt werden muß. Dieser Abtrennungsschritt verursacht Abfallströme, die aufwendig aufgearbeitet werden müssen. Außerdem läßt sich der homogene Katalysator nicht regenerieren.

Die beschriebenen Nachteile bestehen bei der heterogen katalysierten Olefinoligomerisierung nicht. Die wichtigsten industriell ausgeübten heterogen katalysierten Olefinoligomerisierungsverfahren sind z.B. in A. Chauvel und G. Lefebvre, Petrochemical Process, Edition Technip (1989), S. 183-187 und F. Asinger, Die petrolchemische Industrie, Akademie-Verlag (1971), S. 278-299 angegeben.

Die Oligomerisierungsreaktion am heterogenen Katalysator verläuft exotherm. Aufgrund der niedrigeren Investitionskosten ist man bestrebt, die Olefinoligomerisierung in adiabatisch betriebenen Reaktoren durchzuführen. Unter adiabatischer Reaktionsführung wird im Unterschied zur isothermen Reaktionsführung, bei der die bei einer exothermen Umsetzung entstehende Reaktionswärme durch Kühlung mittels Kühl- oder Thermostatiervorrichtungen abgeführt und so die Temperatur im Reaktor konstant, d.h. isotherm, gehalten wird, eine Betriebsweise verstanden, bei der die in einer exothermen Reaktion freiwerdende Wärmemenge nahezu aussschließlich von der Reaktionsmischung im Reaktor aufgenommen und keine Kühlung durch Kühlvorrichtungen angewandt wird. Bei der adiabatischen Fahrweise erfolgt der Abtransport sowohl der gebildeten Reaktionsprodukte als auch der Reaktionswärme durch den durch die Reaktionszone geleiteten Strom des Reaktionsgemisches. Die Temperatur des Kohlenwasserstoffstroms steigt daher entlang des Reaktors mit fortlaufendem Reaktionsfortschritt an. Die Temperaturdifferenz zwischen Austritts- und Eintrittstemperatur des Reaktionsgemisches wird bisweilen als Temperaturtönung bezeichnet.

Eine Möglichkeit der Verfahrenssteuerung besteht in der Regelung der Eintrittstemperatur des Kohlenwasserstoffgemisches. Aus Gründen der Energieersparnis ist eine Temperatur des eintretenden Kohlenwasserstoffgemisches bevorzugt, die möglichst nahe bei Umgebungstemperatur liegt. Andererseits muss die Eintrittstemperatur ausreichend hoch sein, um die gewünschte Katalysatoraktivität und Reaktionsgeschwindigkeit und somit den angestrebten Umsatz zu gewährleisten. Weiter ist die mit zunehmender Betriebsdauer nachlassende Katalysatoraktivität zu berücksichtigen. Üblicherweise wird beim Nachlassen der Katalysatoraktivität die Eintrittstemperatur höher gewählt, um eine höhere Reaktionsgeschwindigkeit zu erreichen und damit die mit der Zeit abnehmende Katalysatoraktivität zu kompensieren. Die Eintrittstemperatur kann allerdings nicht beliebig erhöht werden. Die maximale Temperatur, die das Kohlenwasserstoffgemisch beim Passieren der Reaktionszone erreichen darf, ist nach oben durch Sicherheitsaspekte und praktische Erwägungen, wie den Maximaldruck, für den die verwendete Anlage ausgelegt ist, begrenzt. Ist die Katalysatoraktivität soweit zurückgegangen, dass das Kohlenwasserstoffgemisch beim Austritt aus der Reaktionszone notwendigerweise diese maximale Temperatur hat, so ist der Katalysator erschöpft und muss durch neuen Katalysator ersetzt werden. Die maximale Eintrittstemperatur ist also durch die maximal zulässige Reaktoraustrittstemperatur abzüglich der über den Reaktor auftretenden Temperaturtönung festgelegt.

Die ältere Anmeldung DE-197 505 31.7 beschreibt ein Verfahren zur Herstellung von Octenen und Dodecenen durch Oligomerisierung von Buten-1 und/oder Buten-2 und Butan enthaltenden Kohlenwasserstoffströmen über einem Nickel enthaltenden heterogenen Katalysator, wobei man solche Mengen des von dem Reaktionsgemisch abgetrennten Butans und nicht umgesetzten Butens in die Oligomerisierungsreaktion zurückführt, dass die maximale Konzentration an Oligomeren im Reaktionsgemisch 25 Gew.-%, bezogen auf das gesamte Reaktions-Kohlenwasserstoff-Gemisch, nicht übersteigt.

Es ist bereits vorgeschlagen worden, einen adiabatisch betriebenen Festbettreaktor in mehrere Reaktionszonen zu unterteilen, wobei das Reaktionsgemisch zwischen den einzelnen Reaktionszonen durch indirekten Wärmetausch oder durch Kaltgaseinmischung gekühlt wird, vgl. z.B. M. Baerns (Hrsg.) Chem. Reaktionstechnik, Thieme-Verlag, 1987, S. 249.

Die ältere Anmeldung DE 199 15 357.4 beschreibt ein Verfahren zur Oligomerisierung von $C_2$-$C_8$-Olefinen an einem Nickel enthaltenden heterogenen Katalysator, wobei der Katalysator vor dem Inkontaktbringen mit dem Einsatzkohlenwasserstoffgemisch vorbehandelt wird, indem er mit einem demgegenüber olefinärmeren Kohlenwasserstoffgemisch in Kontakt gebracht wird. Es ist ausgeführt, dass die Betriebsphase in einer Reaktorkaskade aus zwei oder mehreren Oligomerisierungsreaktoren durchgeführt werden kann, wobei das teilumgesetzte Reaktionsgemisch nach Verlassen des einen Reaktors und vor Eintritt in den nachfolgenden Reaktor der Kaskade gekühlt wird.

Aus der US 4,942,021 geht ein kontinuierliches mehrstufiges Verfahren zur Veredelung eines niedere Olefine enthaltenden Zulaufs hervor, wobei der Zulauf in einem ersten Reaktor über einen sauren Zeolith-Katalysator geleitet wird; der Austrag aus dem ersten Reaktor durch Einspritzen von Wasser gekühlt wird; das Gemisch von Reaktoraustrag und Wasser in einen zweiten Reaktor über einen metallhaltigen Zeolith-Katalysator geleitet wird.

Die US 5,019,357 offenbart ein zweistufiges katalytisches System zur Umwandlung eines Niederolefinzulaufs in schwerere flüssige Kohlenwasserstoffe.

Die in den einzelnen Reaktionszonen auftretende Temperaturtönung ist geringer als in einem einheitlichen Reaktor gleichen Katalysatorvolumens. Der Temperaturbereich, über den die Eintrittstemperatur variiert werden kann, um die nachlassende Katalysatoraktivität zu kompensieren, ist daher bei dieser Anordnung höher. Der Katalysator kann somit über einen längeren Zeitraum verwendet werden, was einen Kostenvorteil darstellt.

Es ist festgestellt worden, dass bei Verwendung Nickel enthaltender Oligomerisierungskatalysatoren bei gleichmässiger Aufteilung des Katalysatorvolumens auf z.B. zwei Reaktionszonen die Temperaturtönung in der ersten Reaktionszone erheblich größer ist als in der zweiten Reaktionszone. Der Temperaturbereich, über den die Eintrittstemperatur variiert werden kann, ist durch die höhere Temperaturtönung in der ersten Reaktionszone begrenzt, da die Maximaltemperatur, die das Kohlenwasserstoffgemisch erreichen darf, dort früher erreicht wird als in der zweiten Reaktionszone. Die obere Temperaturgrenze kann daher in der zweiten Reaktionszone nicht vollständig ausgenutzt werden; der Katalysator in der zweiten Reaktionszone ist noch aktiv, während der Katalysator in der ersten Reaktionszone bereits ausgetauscht werden muss. Die Katalysatoraktivität in der zweiten Reaktionszone kann daher nicht vollständig ausgeschöpft werden.

Der Erfindung liegt daher die Aufgabe zugrunde, ein Verfahren anzugeben, das eine möglichst vollständige Nutzung der Katalysatoraktivität erlaubt.

Es wurde gefunden, dass diese Aufgabe gelöst wird, indem das Katalysatorvolumen so auf die Reaktionszonen verteilt wird, dass die in den einzelnen Reaktionszonen auftretenden Temperaturtönungen möglichst gleich hoch sind.

Gegenstand der Erfindung ist daher ein Verfahren, bei dem ein Strom eines olefinhaltigen Kohlenwasserstoffgemisches nacheinander in n aufeinanderfolgenden adiabatisch betriebenen Reaktionszonen über einen heterogenen, Nickel-oxid enthaltenden Oligomerisierungskatalysator geleitet wird, mit $n \geq 2$, wobei das Kohlenwasserstoffgemisch in jeder Reaktionszone eine Temperaturerhöhung $\Delta T_{Reakt}$ erfährt und das Kohlenwasserstoffgemisch mit einer Temperatur $T_{ein}$ in die erste Reaktionszone eintritt und vor dem Eintritt in jede weitere Reaktionszone auf eine Temperatur gekühlt wird, die $T_{ein}$ um jeweils bis zu 20°C über- oder unterschreiten kann, das dadurch gekennzeichnet ist, dass das Katalysatorvolumen der zweiten und, sofern vorhanden, jeder weiteren Reaktionszone größer ist als das der vorhergehenden und die relativen Katalysatorvolumina der einzelnen Reaktionszonen so bemessen sind, dass beim Vergleich zweier Reaktionszonen die Differenz von $\Delta T_{Reakt}$ höchstens 20°C beträgt.

Die relativen Katalysatorvolumina der einzelnen Reaktionszonen sind demzufolge so bemessen, dass für alle Paare i,j

$$\left| \Delta T_{Reakt}{}^{i} - \Delta T_{Reakt}{}^{j} \right| \leq 20°C$$

wobei $\Delta T_{Reakt}{}^{i}$ und $\Delta T_{Reakt}{}^{j}$ für die Temperaturerhöhung des Kohlenwasserstoffgemisches in der i-ten beziehungsweise j-ten Reaktionszone stehen, $n \geq 2$ ist, i von 2 bis n und j von 1 bis i-1 läuft.

Vorzugsweise ist $\Delta T_{Reakt}{}^{i} - \Delta T_{Reakt}{}^{j}| \leq 10°C$, insbesondere $\leq 5°C$. Mit anderen Worten sollen die relativen Katalysatorvolumina so bemessen sein, dass die in den einzelnen Reaktionszonen auftretenden Wärmetönungen innerhalb eines Temperaturbandes von 20°C, vorzugsweise 10°C, insbesondere 5°C, liegen. Die Anzahl n der Reaktionszonen beträgt vorzugsweise 2 bis 5, insbesondere 2 oder 3.

Um die vorstehende Bedingung zu erfüllen, ist das Katalysatorvolumen der zweiten und - sofern vorhanden - jeder weiteren Reaktionszone im Allgemeinen um etwa 30 bis 60 Vol.-% größer als das der vorhergehenden. Im bevorzugten Fall von zwei Reaktionszonen (n=2) liegt das Verhältnis des Katalysatorvolumens der ersten Reaktionszone zum Katalysatorvolumen der zweiten Reaktionszone vorzugsweise im Bereich von 30:70 bis 45:55 und beträgt insbesondere etwa 40:60. Bei Verwendung von Reaktionszonen mit im Wesentlichen einheitlichem Querschnitt sind entsprechend die zweite und jede weitere Reaktionszone demzufolge länger als die vorhergehende.

Das Kohlenwasserstoffgemisch tritt mit einer Temperatur $T_{ein}$ in die erste Reaktionszone ein. Aufgrund der exothermen Umsetzung in der. Reaktionszone tritt es mit einer höheren Temperatur aus dieser aus. Vor dem Eintritt in eine weitere Reaktionszone wird es auf eine Temperatur innerhalb 20°C, vorzugsweise innerhalb 10°C, insbesondere 5°C, von $T_{ein}$ gekühlt. Das Kühlen des Kohlenwasserstoffgemisches erfolgt vorzugsweise durch indirekten Wärmetausch, üblicherweise durch geeignete Wärmetauscher oder dergleichen. Es können mit Vorteil mehrere in Reihe geschaltete Wärmetauscher verwendet werden, die bei Bedarf zu- oder abgeschaltet werden können. Das Einspritzen von Kühlmitteln oder frischem, olefinhaltigen Zulauf in das teilumgesetzte Kohlenwasserstoffgemisch ist nicht bevorzugt. Weiterhin ist bevorzugt, dass die Zusammensetzung des teilumgesetzten Kohlenwasserstoffgemisches zwischen den Reaktionszonen nicht verändert wird, d.h. es werden vorzugsweise keine Komponenten abgetrennt oder beigemischt.

In der Regel ist $T_{ein}$ umso höher zu wählen, je länger der Katalysator im Einsatz war, um die mit der Betriebszeit abnehmende Katalysatoraktivität zu kompensieren. $T_{ein}$ kann z.B., insbesondere bei Verwendung eines $C_4$-Olefine enthaltenden Kohlenwasserstoffgemisches, im Bereich von 20 bis 120°C variiert werden. Die maximale Austrittstemperatur ist im Wesentlichen durch Sicherheitsaspekte und in bestimmten bevorzugten Ausführungsformen der Erfindung, bei denen mit flüssigen Einsatzgemischen gearbeitet wird, dadurch bestimmt, dass das Kohlenwasserstoffgemisch bei dem gewählten Druck noch in flüssiger Phase vorliegt.

Die Reaktionszonen im erfindungsgemäßen Verfahren werden adiabatisch betrieben. Unter adiabatischer Reaktionsführung wird eine Betriebsweise verstanden, bei der die bei der Oligomerisierung am heterogenen Katalysator entstehende Wärmemenge praktisch vollständig vom Einsatzgemisch abgeführt wird und keine Kühlung durch Kühl- oder Thermostatiervorrichtungen angewandt wird. Es versteht sich, dass ein vernachlässigbar kleiner Teil der bei der exothermen Reaktion freiwerdenden Wärmemenge unvermeidlich auch vom Reaktorkörper aufgenommen und durch Wärmeleitung und -abstrahlung an die Umwelt abgegeben wird. Im technischen Sinne wird deshalb unter einer adiabatischen Reaktionsführung eine Betriebsweise verstanden, bei der, abgesehen von dem durch natürliche Wärmeleitung und -abstrahlung vom Reaktor an die Umgebung abgegebenen Teil der Reaktionswärme, die gesamte Reaktionswärme vom Reaktionsgemisch aufgenommen und mit diesem aus dem Reaktor abgeführt wird.

Die verwendbaren Katalysatoren können unterschiedliche Struktur aufweisen. Es kommen an sich bekannte Katalysatoren in Betracht, wie sie in C.T. O'Connor et al., Catalysis Today, Bd.6 (1990), S.336-338 beschrieben sind. Insbesondere werden trägergebundene Katalysatoren eingesetzt. Die Trägermaterialien können z.B. Kieselsäure, Tonerde, Aluminosilicate, Aluminosilicate mit Schichtstrukturen und Zeolithe, wie Mordenit, Faujasit, Zeolith X, Zeolith-Y und ZSM-5, Zirkoniumoxid, das mit Säuren behandelt ist, oder sulfatiertes Titandioxid sein. Besonders geeignet sind Fällungskatalysatoren, die durch Mischen wässriger Lösungen von Nickelsalzen und Silicaten, z.B. Natriumsilicat mit Nickelnitrat, und gegebenenfalls Aluminiumsalzen, wie Aluminiumnitrat, und Calcinieren erhältlich sind. Geeignete Katalysatoren können auch durch Imprägnieren von Kieselsäure, Tonerde oder Alumosilicaten mit wässrigen Lösungen löslicher Nickelsalze, wie Nickelnitrat, Nickelsulfat oder Nickelchlorid, und anschließende Calcinierung erhalten werden.

Bevorzugt sind Katalysatoren, die im Wesentlichen aus NiO, $SiO_2$, $TiO_2$ und/oder $ZrO_2$ sowie gegebenenfalls $Al_2O_3$ bestehen. Derartige Katalysatoren sind insbesondere bevorzugt, wenn das erfindungsgemäße Verfahren zur Oligomerisierung von Butenen herangezogen wird. Sie führen zu einer Bevorzugung der Dimerisierung gegenüber der Bildung höherer Oligomere und liefern überwiegend lineare Produkte. Am meisten bevorzugt ist ein Katalysator, der als wesentliche aktive Bestandteile 10 bis 70 Gew.-% Nickeloxid, 5 bis 30 Gew.-% Titandioxid und/oder Zirkondioxid, 0 bis 20 Gew.-% Aluminiumoxid und als Rest Siliciumdioxid enthält. Ein solcher Katalysator ist durch Fällung der Katalysatormasse bei pH 5 bis 9 durch Zugabe einer Nickelnitrat enthaltenden wässrigen Lösung zu einer Alkaliwasserglaslösung, die Titandioxid und/oder Zirkondioxid enthält, Filtrieren, Trocknen und Tempern bei 350 bis 650°C erhältlich. Zur Herstellung dieser Katalysatoren wird im einzelnen auf die DE-4339713 verwiesen.

Der Katalysator liegt vorzugsweise in stückiger Form, z.B. in Form von Tabletten, z.B. mit einem Durchmesser von 2 bis 6 mm und einer Höhe von 3 bis 5 mm, Ringen mit z.B. 5 bis 7 mm Außendurchmesser, 2 bis 5 mm Höhe und 2 bis 3 mm Lochdurchmesser, oder Strängen unterschiedlicher Länge eines Durchmessers von z.B. 1,5 bis 5 mm, vor. Derartige Formen werden auf an sich bekannte Weise durch Tablettierung oder Extrusion, meist unter Verwendung eines Tablettierhilfsmittels, wie Graphit oder Stearinsäure, erhalten.

Es ist möglich, in den einzelnen Reaktionszonen unterschiedliche Oligomerisierungskatalysatoren einzusetzen, obgleich die Anwendung des gleichen Katalysators in sämtlichen Reaktionszonen bevorzugt ist.

Bei dem eingesetzten olefinhaltigen Kohlenwasserstoffgemisch handelt es sich in der Regel um ein im Wesentlichen aus Olefinen und gesättigten Kohlenwasserstoffen bestehendes Gemisch, obgleich auch reine Olefinströme verwendet werden können. Das Gemisch enthält vorzugsweise 50 bis 100 Gew.-%, insbesondere 60 bis 100 Gew.-%, $C_2$- bis $C_8$-Olefine, vorzugsweise $C_4$- bis $C_6$-Olefine, insbesondere Butene. Bei Einsatz von $C_4$-Kohlenwasserstoffgemischen werden erfolgreich solche mit einem Olefingehalt von 50 bis 95 Gew.-%, insbesondere 60 bis 90 Gew.-%, verwendet. Die Olefinfraktion kann ein einzelnes Olefin, wie n-Hexen, Propylen, oder ein Gemisch isomerer Olefine, wie isomere Butene, oder ein Gemisch von Olefinen unterschiedlicher Kohlenstoffzahl, wie Gemische von 3-Hexen und 2-Penten, Propylen und Buten oder Propylen und Ethen, umfassen. Die enthaltenen gesättigten Kohlenwasserstoffe weisen in der Regel die gleiche Kohlenstoffanzahl wie die Olefinfraktion auf. In vielen Fällen ist es im Hinblick auf die Eigenschaften der Oligomerisierungsprodukte bevorzugt, dass im eingesetzten Kohlenwasserstoffgemisch weniger als 5 Gew.-%, insbesondere weniger als 3 Gew.%, bezogen auf die Olefinfraktion, verzweigte Olefine enthalten sind.

Ein bevorzugtes Gemisch enthält 50 bis 95 Gew.-%, vorzugsweise 60 bis 90 Gew.-%, Butene und 5 bis 50 Gew.-%, vorzugsweise 10 bis 40 Gew.-%, Butane. Vorzugsweise umfasst die Butenfraktion weniger als 5 Gew.-%, insbesondere weniger als 3 Gew.-% iso-Buten (bezogen auf die Butenfraktion). Die Butenfraktion weist im Allgemeinen folgende Zusammensetzung auf (jeweils bezogen auf die Butenfraktion):

| 1-Buten | 1 bis 99 Gew.-%, |
| cis-2-Buten | 1 bis 75 Gew.-%, |
| trans-2-Buten | 1 bis 75 Gew.-%, |
| iso-Buten | 1 bis 5 Gew.-%. |

Als besonders bevorzugter Einsatzstoff wird das sogenannte Raffinat II verwendet, bei dem es sich um einen iso-Buten-abgereicherten $C_4$-Schnitt aus einer FCC-Anlage oder einem Steamcracker handelt. Raffinat II weist z.B. folgende Zusammensetzung auf:

| i-,n-Butan | 30 Gew.-%; |
| i-Buten | 2 Gew.-% |
| 1-Buten | 35 Gew.-%, |
| trans-2-Buten | 19 Gew.-%, |
| cis-2-Buten | 14 Gew.-%. |

Die großtechnisch zugänglichen Kohlenwasserstoffgemische, die als Einsatzgemische für die Zwecke der vorliegenden Erfindung in Betracht kommen, enthalten oftmals Verbindungen, die als Katalysatorgifte wirken und den Oligomerisierungskatalysator deaktivieren. Hierzu zählen sauerstoffhaltige Verbindungen, wie Alkohole, Aldehyde, Ketone und Ether sowie stickstoffhaltige, schwefelhaltige und halogenhaltige Verbindungen. Die Anwesenheit solcher Katalysatorgifte würde zu einer unerwünschten Verringerung der Katalysatoraktivität führen.

Gemäß einem bevorzugten Aspekt der Erfindung wird das Kohlenwasserstoffgemisch daher vor dem Inkontaktbringen mit dem Katalysator zur Entfernung von Katalysatorgiften über ein Adsorptionsmittel geleitet. Als Adsorptionsmittel sind Molekularsiebe, vorzugsweise mit einem Porendurchmesser von größer als 4 Å bis 15 Å geeignet. Als Molekularsiebe können kristalline, natürliche Aluminiumsilicate, wie z.B. Schichtgittersilicate, wie auch synthetische Molekularsiebe eingesetzt werden. Weiter sind kommerzielle Molekularsiebe, wie z.B. Typen der Fa. Bayer, Dow, Union Carbide, Laporte oder Mobil, geeignet. Die Molekularsiebe können z.B. Zeolithe vom A-, X- und Y-Typ sein. Ferner sind auch synthetische Molekularsiebe geeignet, die neben Silicium und Aluminium als Hauptbestandteile noch andere Atome als Nebenbestandteile aufweisen. Diese können z.B. durch einen Ionenaustausch mit den austauschbaren Kationen in den Zeolithen eingebaut werden. Beispielhaft sei hier der Austausch mit seltenen Erden, wie z.B. Gallium, Indium oder Lanthan, oder mit Nickel, Cobalt, Kupfer, Zink oder Silber, aufgeführt. Darüber hinaus können auch synthetische Zeolithe, in welchen andere Atome, wie z.B. Bor oder Phosphor, die durch Kopräzipitation in das Gitter miteingebaut worden sind, eingesetzt werden.

Weitere geeignete Adsorptionsmittel sind z.B. Aluminiumoxide, Aluminiumphosphate, Siliciumdioxide, Kieselgur, Titandioxide, Zirkondioxide, polymere Adsorbentien und Gemische davon. Das Überleiten des Kohlenwasserstoffgemisches über das Adsorptionsmittel erfolgt zweckmäßigerweise in einem Festbett oder einem Wanderbett. Das Kohlenwasserstoffgemisch kann beim Überleiten über das Adsorptionsmittel in gasförmiger oder flüssiger Phase vorliegen, liegt aber vorzugsweise in flüssiger Phase vor.

Die Konzentration an sauerstoffhaltigen, schwefelhaltigen, stickstoffhaltigen und halogenhaltigen Verbindungen im Kohlenwasserstoffgemisch wird vorzugsweise auf weniger als 1 Gew.-ppm, insbesondere weniger als 0,5 Gew.-ppm, verringert.

Sind Diolefine oder Alkine im Kohlenwasserstoffgemisch vorhanden, werden diese vor der Oligomerisierung vorzugsweise auf weniger als 10 Gew.-ppm, insbesondere weniger als 5 Gew.-ppm, besonders bevorzugt weniger als 1 Gew.-ppm, aus demselben entfernt. Die Entfernung der Diolefine und Alkine kann z.B. durch selektive Hydrierung, z.B. gemäß EP-81041 und DE-1568542, erfolgen.

Die Oligomerisierungsreaktion findet vorzugsweise bei Temperaturen von 30 bis 280°C, insbesondere von 30 bis 140°C und besonders bevorzugt von 40 bis 130°C, und bei einem Druck von 10 bis 300 bar, vorzugsweise von 15 bis 100 bar und insbesondere von 20 bis 70 bar statt. Der Druck wird dabei zweckmäßig so ausgewählt, dass bei der eingestellten Temperatur das Kohlenwasserstoffgemisch flüssig vorliegt.

Bei den Reaktionszonen handelt es sich in der Regel um mit dem Katalysator beschickte zylindrische Reaktoren, die von dem vorzugsweise flüssigen Reaktionsgemisch von oben nach unten oder umgekehrt durchströmt werden. Die Reaktionszonen können auch als Abschnitte in einem einheitlichen Reaktorgehäuse ausgestaltet sein.

Nach dem Verlassen der letzten Reaktionszone werden die gebildeten Oligomere in an sich bekannter weise von den nicht umgesetzten Olefinen und gesättigten Kohlenwasserstoffen getrennt. Die abgetrennten Oligomere können in einem nachfolgenden Fraktionierungsschritt aufgereinigt werden.

In einer bevorzugten Ausführungsform der Erfindung begrenzt man die maximale Konzentration an Oligomeren im Reaktionsgemisch auf 30 Gew.-%, vorzugsweise 25 Gew.-% und insbesondere 22 Gew.-%, bezogen auf das Reaktions-Kohlenwasserstoffgemisch. In der Regel wird eine untere Grenze von 10 Gew.-% Oligomeren im umgesetzten Reaktionsgemisch vor dessen Aufarbeitung nicht unterschritten. Durch diese Vorgehensweise kann die Selektivität des erfindungsgemäßen Oligomerisierungsverfahrens bezüglich linearer Oligomere beträchtlich erhöht und die Katalysatordeaktivierung verzögert werden. Die Begrenzung der Oligomerenkonzentration kann durch eine Verringerung der Verweilzeit in der Reaktionszone, d.h. eine Erhöhung der Strömungsgeschwindigkeit erreicht werden. Hierzu wird mit Vorteil ein Teilstrom des von den gebildeten Oligomeren befreiten, aus der letzten Reaktionszone austretenden Kohlenwasserstoffstroms, der im Wesentlichen aus nicht umgesetzten Olefinen und/oder gesättigten Kohlenwasserstoffe besteht, in die erste Reaktionszone zurückgeführt. Das Gewichtsverhältnis von Rückführstrom zu frisch zugeführtem Einsatzkohlenwasserstoffstrom beträgt z.B. 0,5 bis 10, vorzugsweise 1 bis 7, besonders bevorzugt 1 bis 4, wobei sich diese Werte auf den stationären Zustand des Reaktionssystems beziehen.

Es hat sich gezeigt, dass bei der bevorzugten Begrenzung des maximalen Oligomerenanteils im Reaktions-Kohlenwasserstoffgemisch auf Werte von weniger als 30 Gew.-% vor allem bei niedriger Konzentration der Olefine im Einsatz-Kohlenwasserstoffgemisch das zur Erreichng hoher Olefinumsätze, z.B. von 95% und mehr, erforderliche Katalysatorvolumen bisweilen sehr groß wird. Man hat gefunden, dass das Gesamtkatalysatorvolumen deutlich reduziert werden kann, wenn das Oligomerisierungsverfahren in einer zweistufigen Kaskade durchgeführt wird, wobei nach jeder Stufe ein Teil des von den in der jeweiligen Stufe gebildeten Oligomeren befreiten Kohlenwasserstoffstroms vor die jeweilige Stufe zurückgeführt wird.

Die Erfindung wird durch die beigefügte Figur und das folgende Beispiel näher veranschaulicht.

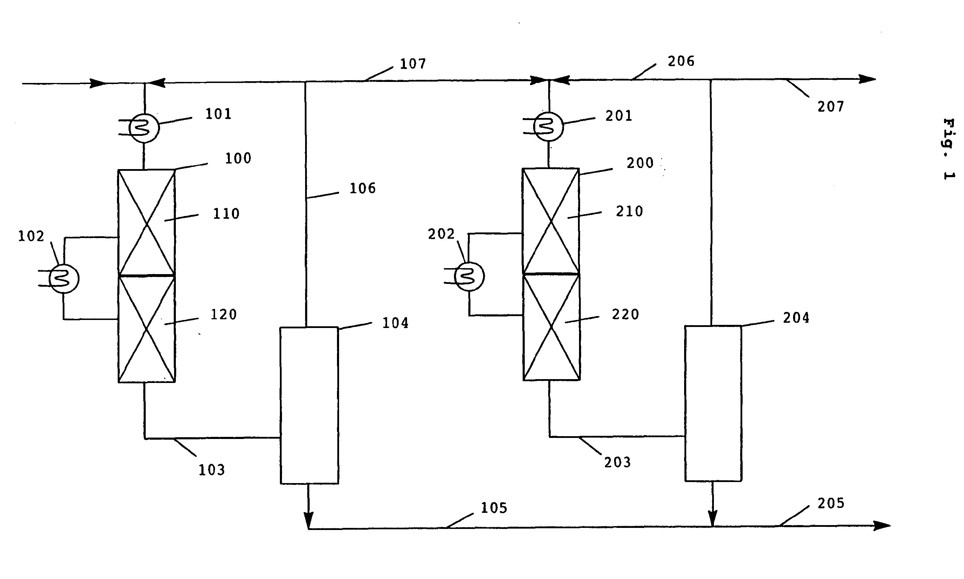

Fig. 1 zeigt schematisch eine zur Durchführung des erfindungsgemäßen Verfahrens geeignete Vorrichtung. Die Vorrichtung umfasst zwei Reaktionsstufen (100) bzw. (200), die jeweils aus zwei Reaktionszonen bestehen (110; 120) bzw. (210; 220). Zwischen den beiden Reaktionszonen wird das Reaktionsgemisch jeweils in den Wärmetauschern (102) bzw. (202) gekühlt. In den Wärmetauschern (101) bzw. (201) wird das Kohlenwasserstoffgemisch auf die jeweilige Eintrittstemperatur gebracht. Nach dem Verlassen der ersten Reaktionsstufe wird das Reaktionsgemisch über die Leitung (103) zur Destillationskolonne (104) geleitet, wo die gebildeten Oligomere abgetrennt werden und über die Leitung (105) abgeführt werden. Die unumgesetzten Olefine und gesättigten Kohlenwasserstoffe werden an der Leitung (106) abgenommen und teilweise in die erste Stufe zurückgeführt und teilweise über die Leitung (107) in die zweite Reaktionsstufe geleitet. Das Verhältnis des zurückgeführten Stroms zum in die zweite Stufe geleiteten Strom ist so gewählt, dass am Ausgang des Reaktors (100) eine vorgegebene Konzentration an Oligomeren nicht überschritten wird. Das Reaktionsgemisch der zweiten Stufe wird in der Destillationskolonne (204) in gebildete Oligomere und unumgesezte Olefine bzw. gesättigte Kohlenwasserstoffe aufgetrennt. Letztere werden über die Leitung (206) teilweise in die zweite Stufe zurückgeführt und teilweise über die Leitung (207) aus dem Verfahren ausgeleitet. Das Verhältnis des zurückgeführten Stroms zum ausgeleiteten Strom ist so gewählt, dass am Ausgang des Reaktors (200) eine vorgegebene Konzentration an Oligomeren nicht überschritten wird.

Beispiel: Mathematische Simulation eines Olefinoligomerisierungsverfahrens

Die Umsetzung eines Einsatzkohlenwasserstoffgemisches der nachstehenden Zusammensetzung an einem Katalysator, der gemäß DE-4339713 in Form von Tabletten mit den Abmessungen 5 mm x 5 mm hergestellt worden war (Zusammensetzung in Gew.-% der Aktivkomponenten: NiO 50 Gew.-%, $TiO_2$ 12,5 Gew.-%, $SiO_2$ 33,5 Gew.-%, $Al_2O_3$ 4 Gew.-%), wurde mittels der Aspen plus-Software (Release 9.3) der Fa. Aspen Tech, Stanford, simuliert. Die Berechnungen beruhen auf einem kinetischen Modell der Umsetzung, das erstellt worden ist, indem eine Vielzahl experimenteller Meßergebnisse mathematisch angepasst wurde.

Es wurde das Katalysatorvolumen berechnet, das bei 1-stufiger Fahrweise bzw. 2-stufiger Fahrweise mit unterschiedlicher Aufteilung des Gesamtumsatzes auf die beiden Stufen (4:1 beziehungsweise 2:1) zur Erlangung eines Gesamtumsatzes von 95% an Octenen und Dodecenen, bezogen auf den Butengehalt des eingesetzten Gemisches, erforderlich ist. Das Katalysatorvolumen bei 1-stufiger Fahrweise wurde willkürlich gleich 100% gesetzt. Es wurden Rückführströme zugrunde gelegt, die zu einer Maximalkonzentration der in der jeweiligen Reaktionsstufe gebildeten Oligomere im Reaktionsgemisch von 20 Gew.-% führen. Die Ergebnisse sind in der nachstehenden Tabelle zusammengefasst.

Zusammensetzung des Einsatzkohlenwasserstoffgemisches:

| | |
|---|---|
| i-Buten | 0 Gew.-% |
| i-Butan | 4,87 Gew.-%; |
| 1-Buten | 17,15 Gew.-%, |
| cis-2-Buten | 16,14 Gew.-%, |
| trans-2-Buten | 42,37 Gew.-%, |

(fortgesetzt)

| | |
|---|---|
| n-Butan | 19,48 Gew.-%. |

Tabelle: Relative Katalysatorvolumina zur Erreichung eines 95%-igen Umsatzes

| | |
|---|---|
| 1-stufige Fahrweise | 100% |
| 2-stufige Fahrweise 4:1 | 75% |
| 2-stufige Fahrweise 2:1 | 55% |

**Patentansprüche**

1. Verfahren zur Oligomerisierung von $C_2$-$C_8$-Olefinen, bei dem ein Strom eines olefinhaltigen Kohlenwasserstoffgemisches nacheinander in n aufeinanderfolgenden adiabatisch betriebenen Reaktionszonen über einen heterogenen, Nickeloxid enthaltenden Oligomerisierungskatalysator geleitet wird, mit $n \geq 2$, wobei das Kohlenwasserstoffgemisch in jeder Reaktionszone eine Temperaturerhöhung $\Delta T_{Reakt}$ erfährt und das Kohlenwasserstoffgemisch mit einer Temperatur $T_{ein}$ in die erste Reaktionszone eintritt und vor dem Eintritt in jede weitere Reaktionszone auf eine Temperatur gekühlt wird, die $T_{ein}$ um jeweils bis zu 20°C über- oder unterschreiten kann, **dadurch gekennzeichnet, dass** das Katalysatorvolumen der zweiten und, sofern vorhanden, jeder weiteren Reaktionszone größer ist als das der vorhergehenden und die relativen Katalysatorvolumina der einzelnen Reaktionszonen so bemessen sind, dass beim Vergleich zweier Reaktionszonen die Differenz von $\Delta T_{Reakt}$ höchstens 20°C beträgt.

2. Verfahren nach Anspruch 1, wobei die Differenz von $\Delta T_{Reakt}$ höchstens 10°C beträgt.

3. Verfahren nach Anspruch 1 oder 2, wobei $T_{ein}$ umso höher zu wählen ist, je länger der Katalysator im Einsatz war.

4. Verfahren nach einem der vorhergehenden Ansprüche, wobei zwei Reaktionszonen verwendet werden und das Verhältnis des Katalysatorvolumens der ersten Reaktionszone zum Katalysatorvolumen der zweiten Reaktionszone im Bereich von 30:70 bis 45:55 liegt.

5. Verfahren nach einem der vorhergehenden Ansprüche, wobei $T_{ein}$ im Bereich von 20 bis 120°C liegt.

6. Verfahren nach einem der vorhergehenden Ansprüche, wobei man die maximale Konzentration an Oligomeren im Reaktionsgemisch auf 30 Gew.-%, bezogen auf das Reaktions-Kohlenwasserstoffgemisch begrenzt.

7. Verfahren nach Anspruch 6, wobei ein Teilstrom des von den gebildeten Oligomeren befreiten aus der n-ten Reaktionszone austretenden Kohlenwasserstoffstroms in die erste Reaktionszone zurückgeführt wird.

8. Verfahren nach einem der vorhergehenden Ansprüche, wobei der Kohlenwasserstoffstrom in flüssiger Phase vorliegt.

**Claims**

1. A process for the oligomerization of $C_2$-$C_8$-olefins, in which a stream of an olefin-containing hydrocarbon mixture is passed over a heterogeneous, nickel oxide-containing oligomerization catalyst in n successive adiabatically operated reaction zones, where $n \geq 2$, and the hydrocarbon mixture experiences a temperature increase $\Delta T_{react}$ in each reaction zone and the hydrocarbon mixture enters the first reaction zone at a temperature $T_{in}$ and before entering each further reaction zone is cooled to a temperature which in each case may be up to 20°C above or below $T_{in}$, wherein the catalyst volume of the second and, if present, each further reaction zone is greater than that of the preceding zone so that the difference in $\Delta T_{react}$ between any two reaction zones is not more than 20°C.

2. A process as claimed in claim 1, wherein the difference in $\Delta T_{react}$ is not more than 10°C.

3. A process as claimed in claim 1 or 2, wherein $T_{in}$ is higher, the longer the catalyst has been used.

4. A process as claimed in any of the preceding claims, wherein two reaction zones are used and the ratio of the catalyst volume of the first reaction zone to the catalyst volume of the second reaction zone is in the range from 30: 70 to 45:55.

5. A process as claimed in any of the preceding claims, wherein $T_{in}$ is in the range from 20 to 120°C.

6. A process as claimed in any of the preceding claims, wherein the maximum concentration of oligomers in the reaction mixture is limited to 30% by weight, based on the hydrocarbon reaction mixture.

7. A process as claimed in claim 6, wherein a substream of the hydrocarbon stream which leaves the $n^{th}$ reaction zone and has been freed of the oligomers formed is recirculated to the first reaction zone.

8. A process as claimed in any of the preceding claims, wherein the hydrocarbon stream is in the liquid state.

**Revendications**

1. Procédé d'oligomérisation d'oléfines en $C_2$-$C_8$, dans lequel un courant d'un mélange d'hydrocarbures contenant de l'oléfine est conduit dans n zones réactionnelles successives, mises en service de manière adiabatique, sur un catalyseur d'oligomérisation hétérogène, contenant de l'oxyde de nickel, où $n \geq 2$, dans lequel le mélange d'hydrocarbures subit dans chaque zone réactionnelle une augmentation de température $\Delta T_{réact}$ et le mélange d'hydrocarbures pénètre dans la première zone réactionnelle avec une température $T_e$ et est refroidi, avant l'entrée dans chaque zone réactionnelle ultérieure, à une température qui peut dépasser $T_e$ vers le haut ou vers le bas de chaque fois jusqu'à 20°C, **caractérisé en ce que** le volume de catalyseur de la deuxième zone réactionnelle et, dans la mesure où il y en a, de chaque zone réactionnelle ultérieure est plus grand que celui de la zone réactionnelle précédente et les volumes relatifs de catalyseur des différentes zones réactionnelles sont mesurés de façon que, lors d'une comparaison entre deux zones réactionnelles, la différence de $\Delta T_{réact}$ soit au maximum de 20°C.

2. Procédé suivant la revendication 1, dans lequel la différence de $\Delta T_{réact}$ est au maximum de 10°C.

3. Procédé suivant la revendication 1 ou 2, dans lequel $T_e$ est à choisir d'autant plus élevé que le catalyseur en chargement est plus long.

4. Procédé suivant l'une des revendications précédentes, dans lequel on utilise deux zones réactionnelles et le rapport entre le volume de catalyseur de la première zone réactionnelle et le volume de catalyseur de la deuxième zone réactionnelle est de l'ordre de 30/70 à 45/55.

5. Procédé suivant l'une des revendications précédentes, dans lequel $T_e$ est de l'ordre de 20 à 120°C.

6. Procédé suivant l'une des revendications précédentes, dans lequel on limite la concentration maximale d'oligomères dans le mélange réactionnel à 30 % en poids, par rapport au mélange d'hydrocarbures de la réaction.

7. Procédé suivant la revendication 6, dans lequel un courant partiel du courant d'hydrocarbures exempt des oligomères formés et sortant de la n-ième zone réactionnelle est recyclé dans la première zone réactionnelle.

8. Procédé suivant l'une des revendications précédentes, dans lequel le courant d'hydrocarbures se présente en phase liquide.

Fig. 1

EP 1 177 160 B1